# EUROPEAN PATENT SPECIFICATION

(11) **EP 0 331 452 B1**
(45) Date of publication and mention of the grant of the patent: **25.08.1993**
(21) Application number: 89302013.1
(22) Date of filing: 28.02.1989
(51) Int. Cl.: A61M 5/32

(54) **Safety needled medical devices**
Medizinische Vorrichtung mit geschützter Nadel
Dispositif médical avec protection d'aiguille

(30) Priority: 01.03.1988 US 162569; 27.07.1988 US 224920; 27.01.1989 US 303588
(43) Date of publication of application: 06.09.1989
(73) Proprietor: RYAN MEDICAL INC., Brentwood Tennessee 37027 (US)
(72) Inventor: Ryan, Dana W., Franklin, TN (US); Kaiser, James M., Brentwood, TN (US); Hinsch, Robert M., Brentwood, TN (US)
(74) Representative: Austin, Hedley William

(56) References cited:
- EP-A- 0 250 104
- US-A- 4 666 435
- US-A- 4 681 567
- US-A- 4 738 663
- US-A- 4 747 837

## Description

The present invention generally relates to improvements in safety needled medical devices which are designed to minimize the incidence of accidental pricking of the skin and resulting spread of infectious diseases by an exposed contaminated needle after use thereof. The disclosed devices may be used as blood collection tube holders, syringes with or without attached needle, prefilled syringes, and IV catheters.

Accidental needlesticks have long been a problem in the medical profession. Accidental needlesticks most often occur during the recapping of a contaminated needle or immediately after use and prior to safe disposal. Such needlesticks place the medical professional (clinician) at risk. When needles are not recapped, additional accidental needlesticks are caused by uncapped needles found in patient beds, linens, and in garbage cans, and place health care housekeeping and sanitation personnel at risk. Because accidental needlesticks can now result in deadly incurable diseases as well as the previously appreciated serious, but usually curable diseases, the need for eliminating the needlestick problem has reached extreme urgency. In addressing the urgency, many devices have been proposed. Indeed, the prior art discloses a number of devices which are arranged to shield the needle of the device after use, but none are as simple to manufacture, assemble, and use as the devices of the present invention. A benefit of the devices of the present invention is that the devices require no change in the method of use or technique by medical personnel, i.e. the medical practioners will use the devices in the same way they previously used standard hypodermic syringes, blood collection tube holders, etc., except that after use they will move a shield to cover the exposed contaminated needle in a very easy, simple, and straightforward manner, requiring the use of only one hand.

Included in the prior art among many safety devices are safety-needled syringes such as are disclosed in U.S. Patent Nos. 2,571,653 to Bastien, 4,0̸26,287 to Haller, 4,425,120̸ to Sampson et al., 4,573,976 to Sampson et al., 4,631,0̸57 to Mitchell, 4,643,199 to Jennings, Jr. et al., 4,655,751 to Harbaugh, 4,666,435 to Braginetz, 4,681,567 to Masters et al., 4,70̸2,738 to Spencer, 4,70̸2,739 to Milorad, 4,723,943 to Spencer, 4,737,144 to Choksi, 4,738,60̸3 to Bogan, 4,747,830̸ to Gloyer et al, 4,747,837 to Hauck, and 4,758,231 to Haber et al. None of these devices, however, have yet gained acceptance in the medical field. Many of the devices require complex pieces or are of such a design such that they are expensive to manufacture and assemble. Others of the devices require the clinician's procedure and technique to change. Yet other devices, while relatively simple in construction and use, do not provide the required level of safety desirable from a "safety" needled device.
For example, the device disclosed in EP-A-0250104 comprises a medical device for assembly with a hollow needle, the medical device having a hollow inner tube member and a telescoping plastic outer shield member, the inner tube member having a front end adapted to have the hollow needle secured thereto, an open rear end, and an outer surface having first and second substantially circumferential grooves, the first groove being forward of the second groove, the inner tube member having a shoulder on its outer surface adjacently forward of the first groove, and the outer shield member having a front end having an opening therein, a substantially open rear end, and at least one inwardly extending protrusion located along the inner surface of the outer shield member, each of the at least one inwardly extending protrusions adapted for engaging at least one of the first groove and the second groove such that when an inwardly extending protrusion engages the second groove, the shield member is maintained in a first retracted position, and when an inwardly extending protrusion engages the first groove, the shield member is locked in a second extended position in which the needle is protected by the shield member, wherein the inwardly extending protrusion engaging the second groove is disengageable from the second circumferential groove, and the shield member is slidable between the first position and the second position.

A major drawback of the device disclosed in EP-A-0250104 is that manufacturing assembly, of the device is extremely difficult, since when sliding the outer shield over the inner tube, the inwardly extending protrusion inevitably locates in the receiving groove. In order that the protrusion can be slid past the groove on assembly, the protrusion is provided with sloping surfaces such that when extreme force is applied the protrusion may "ride" over the sides of the groove. This construction however inevitably entails than when in the extended position covering the used needle, the protrusion is not securely located in the groove, and the outer shield may accidentally be pulled off the inner tube exposing the contaminated needle tip.

It is an object of the present invention to improve on devices such as that referred to above. The improvement is achieved by providing that the shoulder on the outer surface of the inner tube immediately adjacently forward of the groove is slightly larger in diameter than the outer surface of the inner tube member adjacently rearward of the first groove, wherein the inner diameter of the inwardly extending protrusion engaging the first groove is smaller than the outer diameter of the shoulder and smaller than the outer diameter of the outer surface of the inner tube member directly rearwardly adjacent the first groove, such that the inwardly extending protrusion of the outer shield member and the outer surface of the inner tube member are adapted whereby on assembly of the outer shield member on the inner tube member, the inwardly extending protrusion engages the shoulder of the outer surface of the inner tube member and flexes outwardly as a result of the engagement, and subsequently engages the outer surface of the inner tube member rearward of the first groove without fixedly engaging the first groove.

At least one of the grooves is preferably formed towards the rearmost end of the tube adjacent an outward extending finger positioning flange, and at least one other groove preferably being near the forward end of the tube. The rearmost groove is basically a ramp which decreases in diameter as it extends rearward. From the top of the ramp towards the front groove, the outer surface of the inner tube first decreases in diameter, levels off, and then increases in diameter (forming a second ramp) until terminating in the front groove. Thus, a valley is formed between two ramps. Adjacently forward the front groove is a shoulder of slightly greater diameter than the diameter of the outer surface adjacently rearward of the front groove.

The outer safety shield )hereinafter referred to as the "shield", or the "outer shield") is of slightly larger diameter than the inner tube (including very slightly larger than the front shoulder), and is arranged to be slidable relative to the inner tube. The outer shield preferably includes two circumferential inward protrusions in relative close proximity one to the other toward the rear end of the outer shield. The protrusions have an inner diameter approximately equal to the outer diameter of the valley of the inner tube. The rearward of the two protrusions is arranged to engage the rear groove of the inner tube when the shield is in a non-shielded retracted position, while the forward of the two protrusions engages the forward groove of the inner tube when the shield is slid forward into a shielding position. The shield then prevents accidental contact with the contaminated needle, and the rearward protrusion of the outer shield acts both to stabilize the shield relative to the inner tube so that the shield cannot be removed, and also as a second safety catch should the user manage to force the first protrusion forwardly out of the locking groove. Ratchet, or other similar means connected with the inner tube and the outer shield are provided to prevent rotation of the outer shield relative to the inner tube when the shield is in its retracted position and the needle is exposed. The shield also includes ramped flanges on its outer surface around the middle portion of the shield. The ramps increase in diameter as they extend forward along the outer surface of the shield and provide a pushing and gripping surface (of opposite direction to the rear flange of the inner tube) for permitting a one-handed manipulation of the medical devices to accomplish shielding.

A better understanding of the improved safety needle medical devices of the present invention, and additional advantages and objects of the invention will become apparent to those skilled in the art upon reference to the detailed description and accompanying drawings.

### BRIEF DESCRIPTION OF THE DRAWINGS

Fig. 1 is a partially broken-away plan view showing the inner tube and outer shield of the invention prior to assembly for use as a medical safety-needled phlebotomy device;
Fig. 2 is an front end view of the inner tube of Fig. 1, taken along line 2-2 of Fig. 1 and showing a ratchet mechanism and a rear flange;
Fig. 3 is an enlarged partial cross-sectional view of the front ramp, front groove, shoulder section of the inner tube;
Fig. 4 is an enlarged partial cross-section view of the rear ramp, rear groove, rear flange section of the inner tube;
Fig. 5 is a rear end view of the outer shield of Fig. 1, taken along line 5-5 of Fig. 1 and showing a ratchet mechanism and gripping ramps;
Fig. 6 is an enlarged partial cross-sectional view of protrusions at the rear end of the outer shield;
Fig. 7 is an elevational view of the gripping ramps on the outer surface of the outer shield;
Figures 8A and 8B are partially broken-away plan views showing the inner tube and outer shield of the safety needled syringe invention in the unshielded and shielded and positions respectively;
Fig. 9A is a plan view of of the inner tube for a prefilled syringe embodiment of the safety needled invention;
Fig. 9B is a plan view of the plunger arm for a prefilled syringe; and
Fig. 10̸ is a plan view of the front of the inner tube, and the rear of the outer shield of a second embodiment of the safety needled invention;

### DETAILED DESCRIPTION OF THE PREFERRED EMBODIMENT

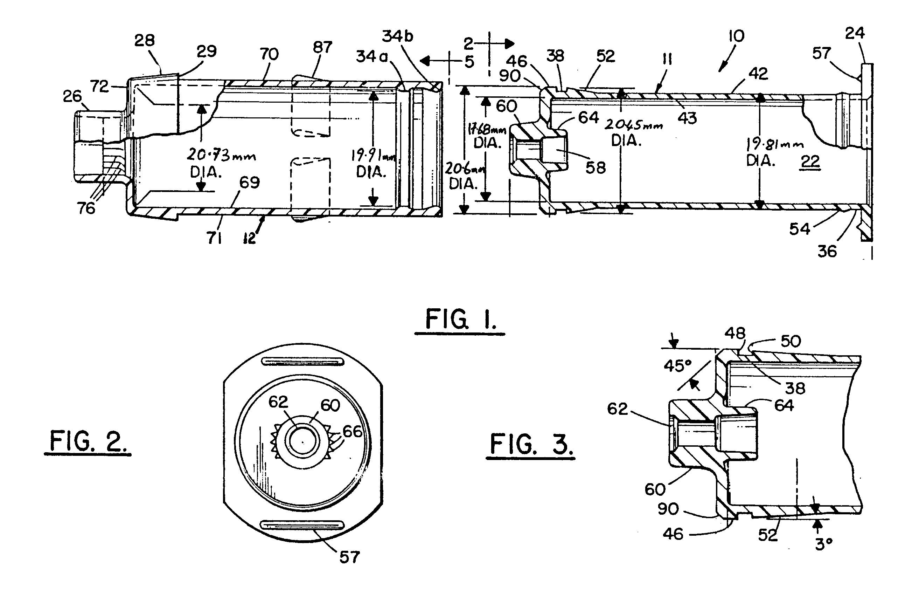

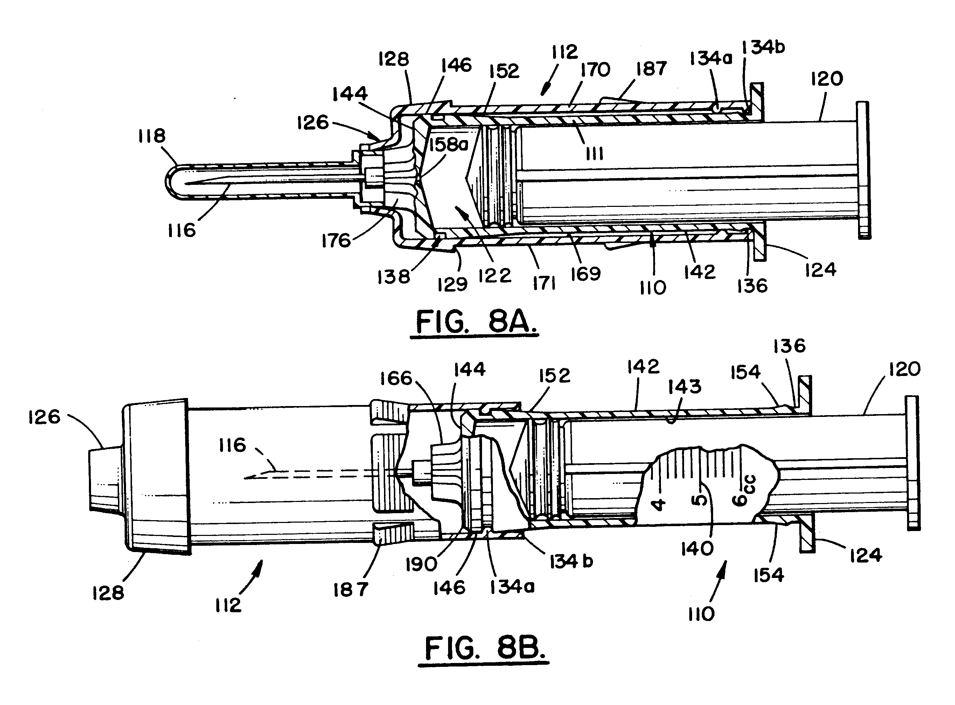

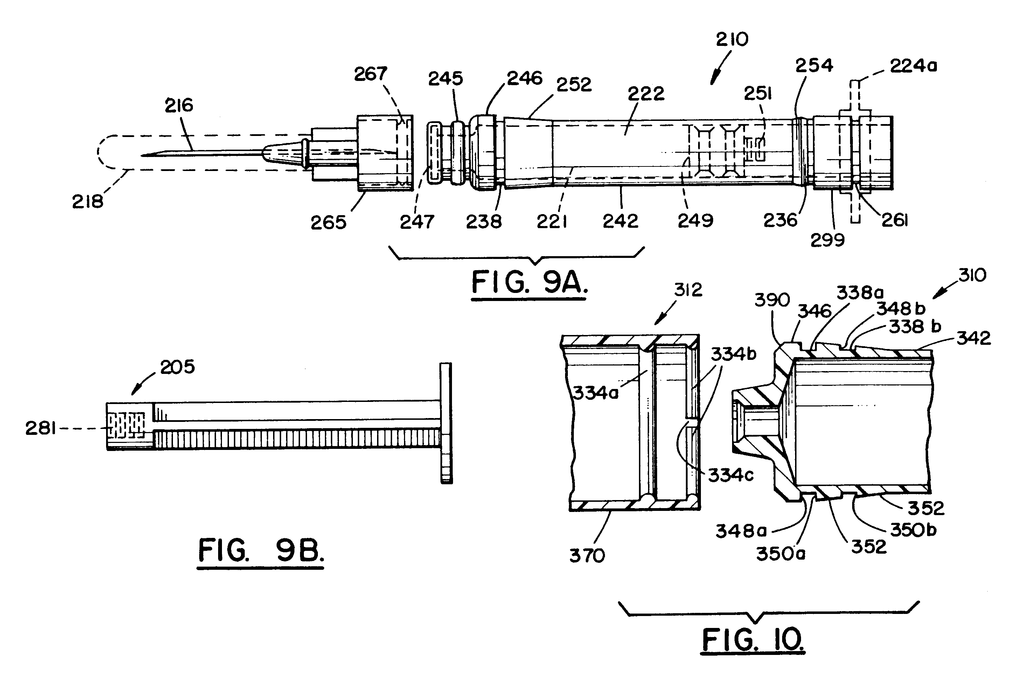

Figure 1-7 generally show the basic structure of the improved safety needled device of the invention although they are particularly directed to a blood collection tube holder. Figures 8A and 8B and Figures 9A and 9B are directed to the syringe and prefilled syringe embodiments respectively, while Figure 10̸ shows a second embodiment of the basic structure.

Referring to Figs. 1-7, the improved safety medical device is seen comprising two generally cylindrical pieces: a hollow inner tube 10̸ and a hollow outer shield 12. Both pieces are preferably formed via injection molding of polypropylene, although other materials of similar properties could be utilized. Looking at detail at the inner tube 10̸ as is best seen in Figures 1-4, the inner surface 43 of the tube is of approximately 17.68 mm (.696 inch) diameter along its entire length to provide a cavity 22 which can accommodate a standard blood collection vacuum tube (not shown). The outer surface 42 of the tube 10̸ along its central portion 11 is of approximately 19.81 mm (.780̸ inch) diameter (approximately the same diameter as the inner surface of the protrusions of the outer shield 12). As best seen in Fig. 3, at the forward end of the outer surface 42 of tube 10̸, a ramp 52 of an approximately three degree angle is formed. When the ramp 52 terminates at forward groove 38 which is bounded by forward ledge 48 and rear ledge 50̸, its outer diameter is approximately 20.45 mm (.80̸5 inches). Directly forward of front groove 38 is a shoulder 46 which must be of slightly greater diameter than the diameter of ramp 52 at its forward termination point. Thus, as shown, the diameter of shoulder 46 is aproximately 20.6 mm (.811 inches). As will be described hereinafter, it is critical that the diameter of shoulder 46 be greater than the diameter of ramp 52 at its forward termination point. It is also strongly preferred that the diameter of shoulder 46 at the same time be smaller than the general inner diameter of the outer shield 12. Since the inner diameter of the provided outer shield is approximately 20.73 mm (.816 inches), the shoulder 46 is preferably no greater tham 20.7 mm (.815 inches) in diameter. As best seen in Fig. 3, the front end 90̸ of shoulder 46 is rounded and angled to permit a more expeditious loading of the outer shield 12 onto the inner tube 10̸ during assembly, as will be described in more detail hereinafter.

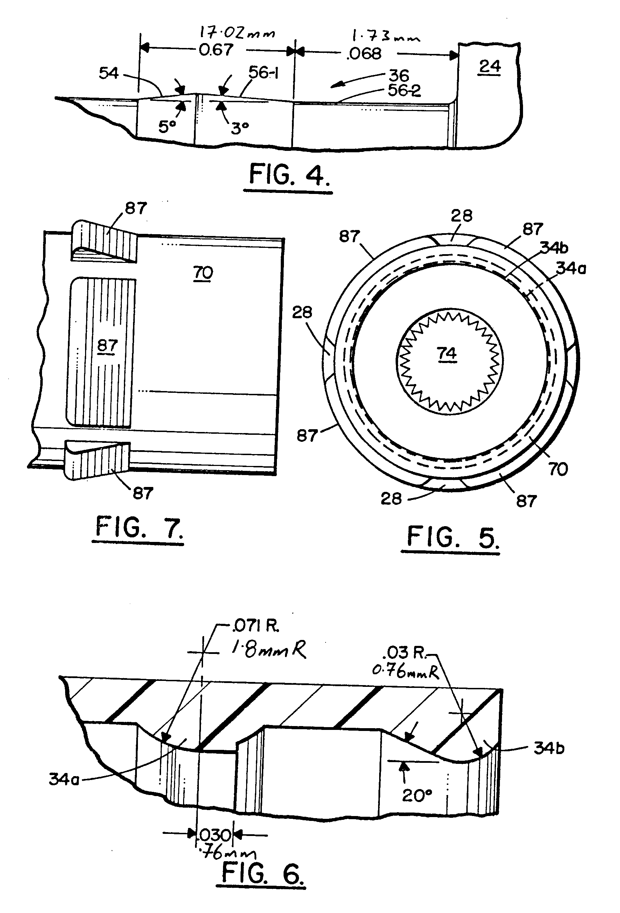

Turning to Fig. 4, the outer surface 42 of the rearward end of inner tube 10̸ is seen in more detail. At the rearward end of the outer surface 42 of tube 10̸, a very short ramp 54 of an approximately five degree angle is formed which increases diameter as it extends rearwardly. When the ramp 54 terminates at rearward "groove" 36 its outer diameter is approximately 19.99 mm (.787 inches). Rearward "groove" 36 then basically comprises a ramp 56-1 at an approximately three degree angle and of decreasing diameter until a 19.81 mm (.780̸) diameter surface is attained, and a small flat portion 56-2. The rear "groove" is defined by the start of ramp 52 and a rear flange 24 which has gripping tines 57 (seen in Fig. 1). As will be described hereinafter, groove 36 serves to keep the outer sleeve 12 in a retracted position to permit blood extraction or fluid injection.

Other aspects of inner tube 10̸ in the blood collection embodiment of Figs. 1-4, include a forwardly extending cylindrical wall 60̸, a rearwardly extending cylindrical wall 64, and a ratchet means 66. The forwardly extending wall 60̸ is provided on its inside circumference with threads 62 or other means by which a standard hollow blood collection needle may be attached so as to communicate through annular opening 58 with cavity 22. Reardwardly extending cylindrical wall 64 is coaxial with the inner tube wall 43 and extending rearwardly from the forward section of the inner tube 10̸. Cylindrical wall 64 is shaped to receive the forward end of a vacuum blood collection vial (not shown) to provide a sealed engagement therebetween. Ratchet means 66, as best seen in Fig. 2, comprises a plurality of teeth which extend radially outwardly from cylindrical wall 60̸. Five teeth 66 are shown on diametrically opposed sides of wall 60̸, but the exact number and exact location of the teeth 66 may be varied. As will be described hereinafter, teeth 66 mesh with reciprocating notches on the shield 12 to prevent rotation of shield 12 relative to inner tube 10̸ when th shield is in its retracted position.

The outer shield 12 is seen best in Figures 1, and 5-7. The outer shield 12 is basically comprised of cylindrical wall 70̸ having inner surface 69 and outer surface 71. With an inner tube 10̸ having a front shoulder of outer diameter 20.6 mm (.811 inches), the diameter of the outer shield inner surface 69 is preferably .816 inches. In fact, the inner diameter of the outer shield 12 preferably is always greater than the largest diameter of any part of inner tube 10̸.

As best seen in Figures 1 and 6, extending inwardly from cylindrical wall 70̸ at the rearward end are circumferential protrusions 34a and 34b. While a single protrusion could be utilized, as will be described hereinafter, the use of two protrusions provides advantages. Protrusions 34a and 34b are preferably uninterrupted, although as seen in Fig. 8a, and Fig. 8b one or both of the protrusions may be divided into a plurality of lugs. Protrusion 34b is arranged to keep shield 12 in a retracted position during use of the needled assembly. Thus, protrusion 34b is arranged to sit in groove 36 of the inner tube 10̸, and is provided with an approximate inner diameter of 19.81 mm (.780̸ inches) which is equal to the outer diameter of groove 36 and valley 11. To permit protrusion 34b to be relatively easily unseated from groove 36 during shielding, protrusion 34b is tapered and rounded as shown. Protrusion 34a is slightly smaller than protrusion 34b (i.e. its approximate inner diameter is 19.91 mm (.784 inches)), and has a rounded forward end and a sheared rear end which provide a locking ledge as will be described in more detail hereinafter.

At the forward end of outer shield 12 an end wall 72 is provided with annular locking nozzle 26 extending therefrom. Besides supporting inwardly extending ratchet means or teeth 76 which are arranged to engage the locking teeth 66 of the inner tube 10̸ as will be described hereinafter, locking nozzle 26 provides guidance during needle attachment and an additional margin of safety against blood leakage and from the needle itself.

Turning to Figures 5 and 7, details of the outer surface of outer shield 12 are shown. Provided around the mid-portion of cylindrical wall 70̸ are ramped flanges 87 which increase in diameter as they extend forward along the outer surface 71 of the shield 12. Ramped flanges 87 provide a pushing and gripping surface for permitting a one-handed manipulation of the medical device as will be described hereinafter. Also formed near the forward end of outer shield 12 on the outer surface thereof is a flange 28 which ramps in the opposite directed to flanges 87. Flange 28 provides a safety ridge or back face 29 to prevent the sliding of the practitioner's hands during use. Also, flange 28 provides a gripping surface for a two-handed manipulation of the medical device.

Figures 8A and 8B show the safety-needled medical device invention in its syringe embodiment with the shield in its retracted (unshielding) and extended (shielded) positions respectively. In Figures 8A and 8B, parts which are identical to or similar to parts of the phlebotomy device of Figures 1-7 are notated by numbers greater by one hundred than the notations of Figures 1-7. Additionally shown in Figures 8A and 8B are the needle assembly 116 which is screwed into the inner tube 110̸, a needle guard 118 which is removed prior to injection, graduated markings 140̸ (typically in cc measurements) which may be located on either the inner shield or outer tube as desired, and a plunger 120̸. Plunger 120̸ is used for either forcing the contents of chamber 122 through a small annular opening 148a and into and through needle 116, or to aspirate a fluid through needle 116, annular opening 158a, and into chamber 22. Annular opening 158a is somewhat different than opening 58 of Figure 1 because annular opening 58 is arranged to receive the vacuum tube as well as connect a needle assembly to chamber 22, while annular opening 158a is a needle sized opening.

As indicated in Figure 8A, prior to use of the medical device, needle cap 118 is located over needle 116. In order to give an injection, the cap is removed, the needle is placed in a medicinal container, plunger 120̸ is drawn backwards to aspirate the medicine, the needle is injected into the patient, and the plunger is pushed forward. During all these procedures, rear projection 134b is seated in rear groove 136 to prevent forward movement of shield 112 relative to inner tube 110̸, and teeth 166 are meshed with ratchet mechanism 176 to prevent rotational movement of shield 112 relative to inner tube 110̸. After injection, the needle is removed from the patient, and the shield is extended into its locked shielding position, as will be discussed hereinafter. In the shielding position, protrusion 134a is fixedly seated in the front groove 138 while protrusion 134b engages ramp 152.

The inner tube 210̸ of the prefilled safety syringe embodiment of the invention is seen in Fig. 9A. (In this embodiment corresponding elements will have numbers corresponding to those of Fig. 1, with the numbers of Fig. 9A being greater by two hundred). The inner tube 210̸ combines many of the standard features of a prefilled syringe with the afore-described inner tube features of the instant safety needled invention. Thus, for purposes of the standard prefilled syringe, the inner tube 210̸ is preferably made of or lined with glass. The medicated liquid 221 is maintained in chamber 222 which is bounded by cylindrical wall 242, a shaped metal cap 245 having a hermetic seal 247, and a rubber plunger seal 249 having a male threaded member 251 extending therefrom. Also, for purposes of the standard prefilled syringe, the rear end 299 of the inner tube 210̸ is provided with a groove 261 for a preferably plastic snap-on flange 224a, while the front end metal cap 245 is arranged to mate with a needle hub 265 having a ridge ring 267 on one end for mating with the metal cap 145 and means for accepting and holding a double pointed needle 216 on the other end. Typically, the needle 216 is provided with a protective cover 218 which must be removed before an injection. A disposable plunger arm 20̸5 seen in Figure 9B is provided with a female thread member 281 which is screwed onto the male threaded member 251 of the inner tube 210̸ prior to injection. After mating, force may be applied to the plunger arm 20̸5 so as to force the medicated liquid out through the double pointed needle 216.

As seen in Figure 9A, the inner tube 210̸ also includes the safety-needled features. Thus, provided in the outer surface of the inner tube 210̸ are front and rear grooves 238 and 236 into which the protrusions of an outer shield may extend. Also, the outer surface of inner tube 210̸ is provided with front shoulder 246, front ramp 252, and rear ramp 254. An outer shield for the prefilled syringe embodiment (not shown) would essentially take the form of shield 12 of Figure 1.

Turning to Figure 10̸ (where elements corresponding to Fig. 1 are denoted by numbers three hundred removed therefrom), additional embodiments of the invention are seen which are applicable to the vacuum tube (phlebotomy) and syringe embodiments. Thus, the rear end of shield 312 is shown with two protrusions 334a and 334b, except that protrusion 334b is segmented by spaces 334c. If desired, either or both of protrusions 334a and 334b can be so segmented. Also, if desired, protrusions 334a and/or 334b could be segmented by providing an incisions or slots (not shown) in the rear of outer shield 312. Also, as seen in Figure 10̸, the inner tube is provided with two front grooves 338a and 338b into which protrusions 334a and 334b may extend. With two front grooves, preferably groove 338a is of a slightly greater width than groove 338b, while protrusion 334a has a slightly greater width than protrusion 334b (and groove 338b). In this manner, during the shielding action, when protrusions 334b of shield 312 are unseated from their rear groove and the shield is moved forward, protrusion 334a will not engage in groove 338b. Rather, protrusion 334a will engage groove 338a, while protrusion 334b will engage groove 338b. Such a double lock could provide additional stability to the apparatus and added security against the shield being moved forward and off the inner tube 310̸.

In manufacturing the safety needled medical device of the invention, the inner tube (except for the prefilled syringe embodiment) and the outer shield are molded out of polypropylene plastic or materials of similar properties. For assembly (for brevity, reference will only be had to the embodiment of Figure 1), the inner tube is then preferably placed upright with its rear end flush with a horizontal surface and some means projecting into the cavity 22 to hold the inner tube upright. The outer shield 12 is correspondingly arranged, except that the outer shield is held from the outside and above the inner tube 10̸. Preferably, just prior to forcing outer shield 12 onto inner tube 10̸, the outer shield 12 is lowered such that protrusion 34b of outer shield 12 engages the angled front 90̸ of shoulder 46 and centers the shield 12 relative to inner tube 10̸. Then, in a sharp downward motion, outer shield 12 is loaded onto inner tube 10̸. The sharp downward motion forces protrusions 34b and 34a repectively over shoulder 46 of inner tube 10̸, thereby instantaneously expanding the resilient shield 12 at the protrusion locations of its rear end. Before the shield 12 can reassume its unstressed position, each protrusion has passed over and by groove 38 and onto ramp 52 of inner tube 10̸. However, because of the outer shield's resilient nature, as the protrusions pass groove 38 they do contract somewhat. Thus, ramp 52 must be provided with a smaller outer diameter than shoulder 46, so that the protrusions will not be nicked or even sheared off in the process. As the shield is further pushed downward over the inner tube, the protrusions slide down ramp 52 and long valley 11. Rear protrusion 34b then rides up ramp 54 and into groove 36 to assume the fully assembled position, while protrusion 34a rests along ramp 54 or on valley 11. In the fully assembled position, the outwardly extending ratchet teeth 66 of inner tube 10̸ engage the ratchet teeth 76 of outer tube 12 to prevent rotation of the shield 12 relative to the inner tube 10̸.

The so-assembled safety medical device may then be used in numerous circumstances and for differing purposes, all of which are within the scope of this invention. A common use would be by a clinician for obtaining blood samples from a patient. For this usage, the clinician screws a capped sterile blood collection needle assembly (similar to that shown in Figure 9A) into the threads 62 of inner tube 10̸. Typically, the phlebotomy needle is pointed on both ends and extends a short distance into cavity 22 of inner tube 10̸. A vacuum vial (not shown) having a rubber or plastic stopper is then inserted into the tube 10̸. The stopper of the vacuum vial is penetrated by contact with the rearward extension of the needle, and blood is drawn into the vacuum vial through the needle which has been inserted into a vein of the patient. Once the blood sample is taken (if desired, several tubes of blood may be obtained), the needle is removed from the patient and the vacuum vial(s) now filled with blood is removed from the inner tube 10̸. The contaminated needle is then shielded by using either a one or two handed method. In the one-handed method, the practitioner might place one or two fingers behind the rear flange 24 of the inner tube, two or three fingers on the shield surface, and the thumb opposite the two or three fingers on the shield surface. Pushing with the thumb, and if desired with additional fingers on either the ramped flange 87, the safety flange 28 or on the outer surface 71 of outer shield 12, the rear protrusion 34b is unseated from rear groove 36 and the shield is slid forward relative to the inner tube 10̸ with the protrusions 34 riding along valley 11 of outer shield 12. When protrusion 34a reaches ramp 52, resistance to the sliding of the shield is met. The practitioner may then move all fingers forwards of rear flange 24, and typically using the thumb and forefinger may press forward on the ramped flange 87 or safety flange 28 so as to force protrusion 34a up ramp 52 and into front groove 38. As the protrusion 34a goes into front groove 38, a click or snap is typically audible. With protrusion 34a engaged in front groove 38, shield 12 completely envelops the needle (in a manner seen in Fig. 8B), thereby rendering it harmless.

In order to permit an easy one-handed manipulation of the safety needled device, the plastic used in the molding process is preferably mixed with a small amount of lubricant (e.g. .25 - .5% in weight). The lubricant included in the resulting molded tube and shield not only eases the practitioner's task of sliding the protrusion of the shield up the ramp and into the circumferential groove, but helps in the molding process by expediting removal of parts from the mold. While a preferred lubricant is a highly purified nitrogenous aliphatic compound, other lubricants can be utilized. Also, a small amount of polyolefin clarifier and colorant can be added to the plastic if so desired to provide a clear tint to the naturally translucent plastic.

In the two-handed technique, the rear flange 24 of inner tube 10̸ is held typically by the forefinger and either the thumb or third finger of one hand, and the shield 12 is held at the ramped flange 87, the safety flange 28 or on the outer surface 71 by the other hand. Then, either by pulling on rear flange 24, pushing on shield 12, or a combination of both, the shield 12 is slid along valley 11, and the protrusion 34a is slid over ramp 54 until it snaps or clicks into front groove 38.

Regardless of the technique used to engage the shield protrusions 34a with the inner tube groove 38 (and regardless of the embodiment; i.e. Figures 1-7, Figures 8A and 8B; Figures 9A and 9B, etc.) it is thereafter extremely difficult to remove the shield from the inner tube. Once protrusion 34a is locked into circumferential groove 38, the front shoulder 46 on the inner tube 10̸ prevents movement of the shield 12 forward, and the forward edge of ramp 52 acts as a second shoulder for preventing movement of the shield backward. Also, the shaped nature of the rear edge of forward protrusion is useful in helping stop rearward movement of the shield. Further, the second protrusion 34b acts to stabilize the assembly and provide an added measure of safety should protrusion 34a somehow become unseated from groove 38 and be moved forward. Thus, with the provided shield and inner tube, a positive lock is assured, completely protecting medical personnel and others against needlestick injuries from contaminated needles. The shielded medical device may then be safely discarded in accord with established procedures.

There has been described and illustrated herein various improved shielded safety medical devices. While particular embodiments of the invention have been described, it is not intended that the invention be limited thereby, as it is intended that the invention be broad in scope. Thus, while particular measurements for the inner tube and shield of the safety phlebotomy device were provided, it will be appreciated that different size phlebotomy and syringe devices can be made within the scope of the invention, provided of course that the shoulder located forwardly adjacent the front groove is larger than the diameter of the ramp rearwardly adjacent the front groove, and preferably just slightly smaller than the inner diameter of the shield, and provided that the inner protrusions on the shield are smaller than the shoulder and preferably smaller than the front ramp diameter. Therefore, it will be apparent to those skilled in the art that yet other changes and modifications may be made to the invention as described without departing from the spirit and scope of the invention as so claimed.

## Claims

1. A medical device for assembly with a hollow needle, the medical device having a hollow inner tube member (10,110,210,310) and a telescoping plastic outer shield member (12,112,312), the inner tube member having a front end adapted to have the hollow needle secured thereto, an open rear end, and an outer surface (11,111) having first (38,138,238,338a) and second (36,136,236) substantially circumferential grooves, said first groove being forward of said second groove, said inner tube member having a shoulder (46,146,246,346) on its outer surface adjacently forward of said first groove, and the outer shield member having a front end having an opening (74) therein, a substantially open rear end, and at least one inwardly extending protrusion (34a,34b,134a,134b,354a,354b) located along the inner surface of said outer shield member, each of said at least one inwardly extending protrusions adapted for engaging at least one of said first groove and said second groove such that when an inwardly extending protrusion engages said second groove, said shield member is maintained in a first retracted position, and when an inwardly extending protrusion engages said first groove, said shield member is locked in a second extended position in which the needle is protected by said shield member, wherein said inwardly extending protrusion engaging said second groove is disengageable from said second circumferential groove, and said shield member is slidable between said first position and said second position, characterised in that said shoulder is slightly larger in diameter than the outer surface (52,152,252,352) of said inner tube member adjacently rearward of said first groove, wherein the inner diameter of said inwardly extending protrusion engaging said first groove is smaller than the outer diameter of said shoulder and smaller than the outer diameter of said outer surface of said inner tube member directly rearwardly adjacent said first groove, such that said inwardly extending protrusion of said outer shield member and said outer surface of said inner tube member are adapted whereby on assembly of said outer shield member on said inner tube member, said inwardly extending protrusion engages said shoulder of said outer surface of said inner tube member and flexes outwardly as a result of the engagement, and subsequently engages said outer surface of said inner tube member rearward of said first groove without fixedly engaging said first groove.

2. A medical device as claimed in claim 1, characterised in that said outer surface of said inner tube member includes a first ramp (52,152,252,352) substantially rearwardly adjacent said first groove, said first ramp being of decreasing diameter as it extends away from said first groove.

3. A medical device as claimed in claim 2, characterised in that said outer surface of said inner tube further includes a second ramp (54,154,254) substantially forwardly adjacent said second groove, said second ramp being of decreasing diameter as it extends away from said second groove, wherein a valley is formed between said first and second ramps.

4. A medical device as claimed in any one of claims 1,2, and 3, characterised in that said inner tube member has a first outwardly extending gripping flange (24,224) substantially at said open rear end thereof, and said plastic outer shield member has a substantially cylindrical outer surface having at least one second gripping flange (87,187) extending outwardly therefrom, such that said outer shield can be slid from said retracted position to said extended position by applying relative forces to first and second flanges.

5. A medical device as claimed in claim 3 and 4, characterised in that said second groove is located substantially adjacent and between said first gripping flange and said second ramp, and said second groove comprises a third ramp, said third ramp (56-1) of increasing diameter as it extends toward said second ramp.

6. A medical device as claimed in any one of the preceding claims characterised in that said at least one inward protrusion comprises at least two generally circumferential protrusions (334a,334b) axially removed along a longitudinal axis of said outer shield, wherein a first of said protrusions is adapted to engage said first groove (338a) in a locking fashion, and a second protrusion is adapted to engage said second groove (338b) in a disengageable fashion.

7. A medical device as claimed in any one of the preceding claims characterised in that said inner tube member further comprises a front end wall having an annular opening and a hollow neck portion (60) supported by said front end wall, said hollow neck portion extending at least a short distance forward of said front end wall and having an opening (62) adjacent said annular opening, said neck portion including outwardly extending locking means (66) about an outer surface of said hollow neck portion, and said shield member further comprises a front end wall (72) and a nozzle (26) supported by said shield member front end wall, said nozzle and said shield member front end wall having openings for permitting the hollow needle to pass therethrough, and said nozzle having inwardly extending locking means (76) located about the inner surface of said nozzle and adapted to lock with said outwardly extending locking means provided on said inner tube member (66) to prevent rotational movement of said shield member relative to said tube member when said shield member is in said retracted position.

8. A medical device as claimed in claim 4, characterised in that said at least one outwardly extending second gripping flange comprises one or both of
at least one ramped gripping flange (87,187) circumferentially located around said shield member and located at other than the extreme rear end and extreme forward end of said shield member, said at least one ramped gripping flange increasing in diameter as it extends toward a forward end of said shield member, said at least one ramped gripping flange arranged to be engaged and pushed by a thumb of a practitioner, and
at least one safety gripping flange (28,128) circumferentially located around said shield member and located at the forward end of said shield member and arranged with a back face (29,129) for gripping between a thumb and a forefinger of a practitioner.

9. A medical device as claimed in any one of the preceding claims characterised in that said inner tube comprises a third groove (338b) proximate to said first groove, said first and third grooves being separated by a distance approximately equal to a distance separating said first (334a) and second (334b) substantially circumferential protrusions, wherein said first groove is wider than said third groove, a first of said circumferential protrusions which is located forward of a second of said circumferential protrusions is of greater axial thickness than said second circumferential protrusion such that said first circumferential protrusion is arranged to lockingly engage said first groove but not said third groove.

10. A medical device as claimed in any one of the preceding claims characterised in that said inner tube member and hollow outer shield member are formed by injection moulding of plastic mixed with a lubricant.

## Patentansprüche

1. Medizinische Vorrichtung für den Zusammenbau mit einer hohlen Nadel, die ein hohles inneres Rohrelement (10, 110, 210, 310) und ein teleskopartig verschiebbares äußeres Kunststoff-Abschirmelement (12, 112, 312) aufweist, wobei das innere Rohrelement ein Stirnende, das für die Befestigung der hohlen Nadel eingerichtet ist, ein offenes hinteres Ende und eine Außenfläche (11, 111) mit einer ersten (38, 138, 238, 338a) und einer zweiten (36, 136, 236) weitgehend in Umfangsrichtung verlaufenden Rille aufweist, wobei die erste Rille vorwärts von der zweiten Rille liegt, wobei das innere Rohrelement eine Schulter (46, 146, 246, 346) an seiner Außenfläche vorwärts neben der ersten Rille aufweist und das äußere Abschirmelement ein Stirnende mit einer Öffnung (74), ein im wesentlichen offenes hinteres Ende und wenigstens einen sich einwärts erstreckenden Vorsprung (34a, 34b, 134a, 134b, 354a, 354b) besitzt, der sich entlang der Innenfläche des äußeren Abschirmelements befindet, wobei jeder der sich einwärts erstreckenden Vorsprünge für den Eingriff mit wenigstens einer der ersten und zweiten Rille eingerichtet ist, so daß bei einem Eingriff eines sich einwärts erstreckenden Vorsprungs mit der zweiten Rille das Abschirmelement in einer ersten zurückgezogenen Position gehalten wird und bei einem Eingriff eines sich einwärts erstreckenden Vorsprungs mit der ersten Rille das Abschirmelement in einer zweiten ausgefahrenen Lage verriegelt ist, in der die Nadel durch das Abschirmelement geschützt ist, wobei der sich einwärts erstreckende, mit der zweiten Rille in Eingriff stehende Vorsprung von der zweiten in Umfangsrichtung verlaufenden Rille lösbar ist, und wobei das Abschirmelement zwischen der ersten und zweiten Position verschiebbar ist, dadurch gekennzeichnet, daß die Schulter einen geringfügig größeren Durchmesser hat als die Außenfläche (52, 152, 252, 352) des inneren Rohrelements rückwärts neben der ersten Rille, wobei der Innendurchmesser des mit der ersten Rille in Eingriff stehenden, sich einwärts erstreckenden Vorsprungs kleiner als der Außendurchmesser der Schulter und kleiner als der Außendurchmesser der Außenfläche des inneren Rohrelements unmittelbar rückwärts neben der ersten Rille ist, so daß der sich einwärts erstreckende Vorsprung des äußeren Abschirmelements und die Außenfläche des inneren Rohrelements aneinander angepaßt sind, wobei beim Zusammenbau des äußeren Abschirmelements mit dem inneren Rohrelement der sich einwärts erstreckende Vorsprung mit der Schulter der Außenfläche des inneren Rohrelements in Eingriff kommt und als Folge des Eingriffs nach außen federt und anschließend mit der Außenfläche des inneren Rohrelements rückwärts von der ersten Rille in Eingriff kommt, ohne fest in Eingriff mit der ersten Rille zu kommen.

2. Medizinische Vorrichtung nach Anspruch 1, dadurch gekennzeichnet, daß die Außenfläche des inneren Rohrelements eine erste Schräge (52, 152, 252, 352) etwa rückwärts neben der ersten Rille aufweist, wobei die erste Schräge einen abnehmenden Durchmesser in ihrer Ausdehnung von der Rille fort aufweist.

3. Medizinische Vorrichtung nach Anspruch 2, dadurch gekennzeichnet, daß die Außenfläche des inneren Rohrs ferner eine zweite Schräge (54, 154, 254) etwa vorwärts neben der zweiten Rille aufweist, daß die zweite Schräge einen abnehmenden Durchmesser in ihrer Ausdehung von der Rille fort aufweist, wobei zwischen der ersten Schräge und der zweiten Schräge ein Tal gebildet wird.

4. Medizinische Vorrichtung nach einem der Ansprüche 1, 2 und 3, dadurch gekennzeichnet, daß das innere Rohrelement etwa an seinem offenen hinteren Ende einen sich nach außen erstrecken-Greifflansch (24, 224) aufweist, und daß das äußere Kunststoff-Abschirmelement eine im wesentlichen zylindrische Außenfläche mit wenigstens einem zweiten sich nach außen erstreckenden Greifflansch (87, 187) aufweist, so daß die äußere Abschirmung durch Anwendung entsprechender Kräfte auf den ersten und zweiten Flansch von der eingezogenen Position in die ausgefahrene Position geschoben werden kann.

5. Medizinische Vorrichtung nach Anspruch 3 oder 4, dadurch gekennzeichnet, daß die zweite Rille etwa neben und zwischen dem ersten Greifflansch und der zweiten Schräge angeordnet ist, und daß die zweite Rille eine dritte Schräge (56-1) mit zunehmendem Durchmesser in Richtung zur zweiten Schräge umfaßt.

6. Medizinische Vorrichtung nach einem der vohergehenden Ansprüche, dadurch gekennzeichnet, daß der wenigstens eine einwärts gerichtete Vorsprung wenigstens zwei allgemein in Umfangsrichtung verlaufende Vorsprünge (334a, 334b) umfaßt, die einen axialen Abstand entlang der Längsachse der äußeren Abschirmung aufweisen, wobei ein erster dieser Vorsprünge für den Eingriff mit der ersten Rille (338a) in einer Verriegelungsart und ein zweiter Vorsprung für den Eingriff mit der zweiten Rille (338b) in einer lösbaren Art eingerichtet ist.

7. Medizinische Vorrichtung nach einem der vorhergehenden Ansprüche, dadurch gekennzeichnet, daß das innere Rohrelement ferner eine Stirnwand mit einer ringförmigen Öffnung und ein von der Stirnwand getragenes hohles Halsteil (60) enthält, daß sich das hohle Halsteil wenigstens ein kurzes Stück von der Stirnwand vorwärts erstreckt und eine Öffnung (62) neben der ringförmigen Öffnung besitzt, daß das Halsteil sich nach außen erstreckende, um seine Außenfläche herum angeordnete Verriegelungsmittel (66) enthält, und daß das Abschirmelement ferner eine Stirnwand (72) und einen von der Stirnwand des Abschirmelements getragenen Stutzen (26) enthält, wobei der Stutzen und die Stirnwand des Abschirmelements Öffnungen zum Durchtritt der hohlen Nadel aufweisen, und daß der Stutzen sich einwärts erstreckende, um ihre Innenfläche herum angeordnete Verriegelungsmittel (76) aufweist, die zum Verriegeln mit den sich nach außen erstreckenden Verriegelungsmitteln (66) an dem inneren Rohrelement ausgebildet sind, um eine Drehbewegung des Abschirmelements relativ zu dem Rohrelement zu verhindern, wenn sich das Abschirmelement in seiner zurückgezogenen Position befindet.

8. Medizinische Vorrichtung nach Anspruch 4, dadurch gekennzeichnet, daß der wenigstens eine sich nach außen erstreckende Greifflansch umfaßt:
wenigstens einen abgeschrägten Greifflansch (87, 187), der auf dem Umfang des Abschirmelements und anderwo als an dessen äußeren hinteren und äußeren vorderen Ende angeordnet ist, wobei der wenigstens eine abgeschrägte Greifflansch in seinem Durchmesser in Richtung zum vorderen Ende des Abschirmelements zunimmt und so eingerichtet ist, daß er mit dem Daumen eines Praktikers erfaßt und geschoben werden kann, und/oder
wenigstens einen Sicherheitsgreifflansch (28, 128), der auf dem Umfang des Abschirmelements und an dessen vorderen Ende angeordnet und mit einer hinteren Stirnfläche (29, 129) zum Greifen zwischen Daumen und Zeigefinger eines Praktikers versehen ist.

9. Medizinische Vorrichtung nach einem der vorhergehenden Ansprüche, dadurch geknnzeichnet, daß das innere Rohr eine dritte Rille (338b) in der Nähe der ersten Rille aufweist, wobei die erste und dritte Rille einen Abstand voneinander haben, der etwa gleich dem Abstand zwischen dem ersten (334a) und zweiten (334b) im Umfangsrichtung verlaufenden Vorsprung ist, wobei die erste Rille breiter als die dritte Rille ist, wobei ein erster der in Umfangsrichtung verlaufenden Vorsprünge, der sich vorwärts von einem zweiten der in Umfangsrichtung verlaufenden Vorsprünge befindet, eine größere axiale Dicke als der zweite in Umfangsrichtung verlaufende Vorsprung hat, so daß der erste in Umfangsrichtung verlaufende Vorsprung für einen verriegelnden Eingriff mit der ersten Rille aber nicht mit der dritten Rille eingerichtet ist.

10. Medizinische Vorrichtung nach einem der vorhergehenden Ansprüche, dadurch gekennzeichnet, daß das innere Rohrelement und das äußere hohle Abschirmelement durch Spritzgießen einer Mischung von Kunststoff mit einem Schmiermittel gebildet ist.

## Revendications

1. Dispositif médical d'assemblage avec une aiguille creuse, le dispositif médical comportant un élément formant tube intérieur creux (10,11, 210, 310) et un élément forment écran télescopique de protection extérieur en matière plastique (12, 112, 312), l'élément formant tube intérieur comportant une extrémité frontale sur laquelle est fixée l'aiguille creuse, une extrémité arrière ouverte, et une face extérieure (11, 111) comportant une première (38, 138, 238, 338a) et une seconde (36, 136, 236) gorges pratiquement circonférentielles, la première gorge se trouvant en avant de la seconde, ledit élément formant tube intérieur comportant un épaulement (46, 146, 246, 346) sur sa face extérieure adjacente à l'avant de la première gorge, et l'élément formant écran de protection extérieur comportant une extrémité frontale ayant une ouverture (74), une extrémité arrière pratiquement ouverte, et au moins une protubérance qui se prolonge vers l'intérieur (34a, 34b, 134a, 134b, 354a, 354b) située le long de la face intérieure dudit élément formant écran de protection extérieur, chacune des protubérances étant conçue pour coopérer avec chacune des premières et secondes gorges de sorte que lorsqu'une protubérance vient en prise avec ladite seconde gorge, ledit élément formant écran de protection est maintenu dans une première position rétractée, tandis que lorsqu'une protubérance vient en prise avec la première gorge, ledit élément formant écran de protection est verrouillé dans une seconde position d'extension, dans laquelle l'aiguille est protégée par ledit élément formant écran de protection, ladite protubérance qui s'étend vers l'intérieur coopérant avec ladite seconde gorge pouvant en être extraite tandis que ledit élément formant écran de protection coulisse entre ladite première position et ladite seconde position, caractérisé en ce que ledit épaulement est légèrement plus grand en diamètre que la face extérieure (52, 152, 252, 352) dudit élément formant tube intérieur adjacent vers l'arrière de ladite première gorge, en ce que le diamètre intérieur de ladite protubérance qui s'étend vers l'intérieur et qui coopère avec ladite première gorge est plus petit que le diamètre extérieur dudit épaulement, et plus petit que le diamètre extérieur de ladite face extérieure dudit élément formant tube intérieur directement adjacent vers l'arrière de ladite première gorge, de sorte que ladite protubérance qui s'étend vers l'intérieur dudit élément formant écran de protection extérieur et ladite surface extérieure dudit élément formant tube intérieur sont conçus pour cela lors de l'assemblage dudit élément formant écran de protection extérieur sur ledit élément formant tube intérieur, ladite protubérance venant en contact avec ledit épaulement de ladite face extérieure dudit élément formant tube intérieur et fléchissant vers l'extérieur du fait du contact et par conséquent vient en prise avec ladite face extérieure dudit élément formant tube intérieur vers l'arrière de ladite première gorge sans venir en prise de façon fixe avec cette première gorge.

2. Dispositif médical selon la revendication 1, caractérisé en ce que ladite face extérieure dudit élément formant tube intérieur comprend une première rampe (52, 152, 252, 352) pratiquement adjacente à ladite première gorge, en direction de l'arrière, ladite première rampe étant de diamètre dégressif au fur et à mesure qu'elle s'étend à l'opposé de ladite première gorge.

3. Dispositif médical selon la revendication 2, caractérisé en ce que ladite face extérieure dudit tube intérieur comprend de plus une seconde rampe (54, 154, 254) pratiquement adjacente à ladite seconde gorge en direction de l'avant, le diamètre de la seconde rampe étant dégressif au fur et à mesure qu'elle s'étend à l'opposé de ladite seconde gorge, une dépression étant formée entre ladite première et ladite seconde rampe.

4. Dispositif médical selon l'une quelconque des revendications 1, 2 et 3, caractérisé en ce que ledit élément formant tube intérieur comporte une première bride de préhension qui s'étend vers l'extérieur (24, 224) pratiquement située à ladite extrémité arrière ouverte, et en ce que ledit élément formant écran de protection extérieur en matière plastique comporte une surface extérieure pratiquement cylindrique pourvue d'au moins une seconde bride de préhension (87, 187) qui s'étend vers l'extérieur, de sorte que ledit écran de protection extérieur peut coulisser depuis ladite position rétractée jusqu'à ladite position en extension en appliquant des forces relatives à la première et la seconde brides.

5. Dispositif médical selon les revendications 3 et 4, caractérisé en ce que ladite seconde gorge est située de façon pratiquement adjacente entre ladite première bride de préhension et ladite seconde rampe, et en ce que ladite seconde gorge comporte une troisième rampe, (56-1) dont le diamètre croît au fur et à mesure qu'elle s'étend vers ladite seconde rampe.

6. Dispositif médical selon l'une quelconque des revendications précédentes, caractérisé en ce qu'au moins une protubérance vers l'intérieur comporte au moins deux saillies globalement circonférientielles (334a, 334b) qui sont écartées axialement suivant l'axe longitudinal dudit écran extérieur, tandis qu'une première desdites saillies est conçue pour venir en prise avec ladite première gorge (338a) de manière à être verrouillée, et que la seconde saillie est propre à venir en prise avec ladite seconde gorge (338b) de manière démontable.

7. Dispositif médical selon l'une quelconque des revendications précédentes, caractérisé en ce que l'élément formant tube intérieur comprend en outre une cloison d'extrémité frontale pourvue d'une ouverture annulaire et d'un col creux (60) solidaire de ladite cloison d'extrémité frontale, le col creux s'étendant au moins sur une courte distance en avant de ladite cloison, une ouverture (62) étant adjacente à ladite ouverture annulaire, ledit col contenant des moyens de verrouillage (66) qui s'étendent vers l'extérieur autour de la face extérieure dudit col creux, et en ce que ledit élément formant écran de protection comprend une cloison d'extrémité frontale (72) et un nez (26) porté par ladite cloison d'extrémité frontale de l'élément formant écran, ledit nez et ladite cloison d'extrémité frontale de l'élément formant écran de protection comportant des ouvertures pour permettre à l'aiguille de passer ledit nez comportant en outre des moyens de verrouillage (76) qui s'étendent vers l'intérieur situés autour de la face intérieure dudit nez, et conçus pour coopérer avec les moyens de verrouillage (66) qui s'étendent vers l'extérieur disposés sur ledit élément formant tube intérieur, afin d'éviter un mouvement de rotation dudit élément formant écran de protection par rapport audit élément formant tube lorsque ledit élément formant écran de protection se trouve dans ladite position rétractée.

8. Dispositif médical selon la revendication 4, caractérisé en ce que la seconde bride de préhension qui s'étend vers l'extérieur comprend une ou deux desdites brides de préhension inclinée (87, 187) située circonférentiellement autour dudit élément formant écran de protection et située ailleurs qu'à l'extrémité arrière et qu'à l'extrémité avant dudit élément formant écran de protection, ladite bride de préhension inclinée augmentant en diamètre au fur et à mesure qu'elle s'étend vers une extrémité frontale dudit élément formant écran de protection, ladite bride de préhension inclinée étant conçue de manière que le doigt d'un praticien puisse venir en contact avec elle et la pousser tandis qu'une des brides de préhension de sécurité (28, 128) située circonférentiellement autour dudit élément formant écran de protection et disposée à l'extrémité frontale dudit élément formant écran de protection présente une face arrière (29, 129), pour préhension entre le pouce et l'index d'un praticien.

9. Dispositif médical selon l'une quelconque des revendications précédentes, caractérisé en ce que ledit tube intérieur comprend une troisième gorge (338b) proche de ladite première gorge, lesdites première et troisième gorges étant écartée d'une distance approximativement égale à la distance d'écartement desdites première (334a) et seconde 334b) saillies pratiquement circonférentielles, et en ce que ladite première gorge est plus large que ladite troisième gorge tandis qu'une première desdites saillies qui est située en avant d'une seconde desdites saillies est d'une épaisseur axiale, supérieure à celle de ladite seconde saillie, de sorte que ladite première saillie est conçue pour venir se verrouiller dans ladite première gorge mais pas dans ladite troisième.

10. Dispositif médical selon l'une quelconque des revendications précédentes, caractérisé en ce que ledit élément formant tube et ledit élément formant écran de protection extérieur creux sont moulés par injection de matière plastique mélangée avec un agent anti-friction.
